Europäisches Patentamt

(19) European Patent Office

Office européen des brevets

(11) Veröffentlichungsnummer: **0 070 328**
**A1**

(12) **EUROPÄISCHE PATENTANMELDUNG**

(21) Anmeldenummer: 81105731.4

(22) Anmeldetag: 21.07.81

(51) Int. Cl.³: **A 61 L 15/04,** A 61 F 1/00,
A 01 N 1/02, A 61 K 35/12,
C 12 N 5/00

(43) Veröffentlichungstag der Anmeldung: 26.01.83
Patentblatt 83/4

(71) Anmelder: **Theurer, Karl Eugen, Prof.Dr.med,**
**Brunnwiesenstrasse 23, D-7302 Ostfildern 1 (DE)**
Anmelder: **Theurer, Karl Georg, Dr.med.,**
**Brunnwiesenstrasse 23, D-7302 Ostfildern 1 (DE)**

(72) Erfinder: **Theurer, Karl Eugen, Prof.Dr.med,**
**Brunnwiesenstrasse 23, D-7302 Ostfildern 1 (DE)**
Erfinder: **Theurer, Karl Georg, Dr.med.,**
**Brunnwiesenstrasse 23, D-7302 Ostfildern 1 (DE)**

(84) Benannte Vertragsstaaten: **AT BE CH DE FR GB IT LI LU NL SE**

(54) Verwendung von Eihäuten und Nabelschnur in nichtbearbeitetem oder bearbeitetem Zustand.

(57) Die Erfindung betrifft die Verwendung von Eihäuten (Amnion- und Allantoismembranen) und Nabelschnur, allogener und xenogener Herkunft, in nativem, wie auch bearbeitetem Zustand, in der Chirurgie bei Operationen zum Über- oder Abdecken, sowie zur Verstärkung brüchiger Gewebe und zur Vorbeugung von Nahtinsuffizienz, insbesondere an Hohlorganen und parenchymatösen Organen, zur Prophylaxe von Verwachsungen, zur Deckung von Haut- und Schleimhautdefekten, zur Verwendung bei plastischen Operationen zum Ausfüllen von Hohlräumen oder zur Unterfütterung, wie auch zum Belegen von Gelenkflächen. Die Fixierung kann durch Naht oder mit Bioklebern, in Form von Fibrin, Kollagen oder Elastin, erfolgen. Die Eihäute können auf der späteren Auflageseite enzymatisch oder chemisch mazeriert und gegebenenfalls insgesamt durch Bedampfen mit konzentrierter Schwefelsäure im Vakuum bei Raumtemperatur sulfatiert werden. Aus der Nabelschnur werden embryonales Bindegewebe, Blutgefäße und äußere Haut präparativ getrennt angewandt oder weiterverarbeitet.

Eine Adaptation an allogene oder xenogene Histokompatibilitäts-Antigene wird während der Vital-Konservierung durch Zusatz von Organextrakten aus Organ- oder Zellgeweben mit Art- oder Individual-Spezifität des Empfängers erreicht. Dieses Verfahren wird auch zur Adaptation von Zellkulturen und für vorgesehene Organtransplantate verwendet.

0070328

Anmelder:  Prof. Dr.med. Karl Eugen Theurer
           Dr.med. Karl Georg Theurer
           Brunnwiesenstraße 23
           D-7302 Ostfildern 1 (Ruit)
           Bundesrepublik Deutschland

Verwendung von Eihäuten und Nabelschnur in nichtbearbeitetem oder bearbeitetem Zustand.


Beschreibung:

Die Erfindung betrifft die Verwendung von Eihäuten
und Nabelschnur allogener und xenogener Herkunft in
der Chirurgie. Unter Eihäuten sind Amnion und
Allantoismembrane, vorzugsweise von Menschen oder
von Säugetieren zu verstehen. Diese besitzen eine
größere Stabilität für die vorgesehene Aufgabe, als
solche aus Hühner- und anderen Vogeleiern. Doch können auch diese für spezielle Aufgaben verwendet werden.

Die Eihäute sind als Membranen beidseitig mit einer
Epithelschicht ausgestattet. Für die Verwendung in
der Chirurgie, zum flächenhaften Belegen von defekten
Geweben, ist es zweckmäßig, daß diese Schicht auf der
späteren Auflageseite der Häute vorher beseitigt wird
und mesenchymales Gewebe unmittelbar mit dem Gewebe des
Patienten in Berührung kommt; doch können auch die
nativen Eihäute verwendet werden. Die Beseitigung
der Epithelschicht auf der späteren Auflageseite erfolgt durch Einwirkung von proteolytischen Enzymen,
wie z.B. Trypsin, Papain, Pepsin, Kollagenasen, u.a.,
wie auch durch Benetzen mit Säuren, z.B. mit 2%iger
Perchlorsäure oder 1%iger Essigsäure oder Laugen bzw.
die Zellen dissoziierenden Stoffen, z.B. Harnstoff.

Die Anwendung kann gegebenenfalls kombiniert erfolgen. Es muß dabei aber eine Schädigung der Epithelzellschicht der anderen Seite vermieden werden.

Die Art der Mazeration bzw. des Abbaus oder der Dissoziation von Zellschichten durch Enzyme und Chemikalien entspricht den bekannten Methoden der Gewebezüchtung und der Histologie. Sie braucht deshalb nicht im Einzelnen. dargestellt werden (vgl. Karl F. Bauer: Methodik der Zell- und Gewebezüchtung: S. Hirzel Verlag Stuttgart, 1974; B. Mauersberger: Aktuelle Probleme der Zellzüchtung: E. Fischer Verlag, 1971). Nach getaner Wirkung werden die Reagenzien durch intensives Spülen mit gepufferten physiologischen Lösungen, z.B. Tyrode-Lösung oder Ringer-Lösung gewebeschonend beseitigt.

Aus Nabelschnur werden hingegen die äußere Haut durch Längsschnitt gespalten und dann das embryonale Bindegewebe und die Blutgefäße präparativ getrennt. Diese Bestandteile können ebenfalls unmittelbar verwendet werden, oder aber in konservierter Form.

Zur Beseitigung der Epithelschicht der Eihäute, wie auch zur Konservierung derselben und der aus Nabelschnur gewonnenen Haut, werden diese über einem Rahmen ausgebreitet, so daß mindestens 4 gegenüberliegende Seiten fixiert sind. Die Fixierung muß sich der Verkleinerung der Häute durch den Konservierungsvorgang anpassen, so daß Einrisse vermieden werden. Technisch läßt sich dies leicht lösen, indem man durch elastische Züge oder Federn jeweils die gegenüberliegende punktförmige Fixierung beweglich gestaltet.

Die Konservierung erfolgt ebenfalls durch bekannte Methoden der Gewebezüchtung (vgl. oben):

a) In Art der Überlebenskulturen von Organexplantaten durch Verwendung von gepufferten Nährmedien mit Antibiotikazusatz bei Temperaturen zwischen +5°C und Körpertemperatur.

b) Durch gegebenenfalls stufenweises Eingefrieren bei Temperaturen des flüssigen Stickstoffs unter Verwendung von Gefrierschutzmittel, z.B. Glyzerin oder Dimethylsulfoxid (DMSO), mit denen die Gewebe vor dem Tiefgefrieren präpariert werden.

c) Durch Gefriertrocknung (Lyophilisation im Vakuum).

d) Durch Gefriertrocknung mit gleichzeitiger partieller Sulfatierung durch Bedampfen mit Schwefelsäuredämpfen im Vakuum bei Raumtemperatur (vgl. DBP 1090 821 und Europäische Patentanmeldung Nr. 80 106066.6). Dadurch wird gleichzeitig auch Sterilität gegen Viren erreicht.

Die Gewebe werden kurze Zeit vor Ihrer bestimmungsgemäßen Verwendung in gepufferten, physiologischen Lösungen, gegebenenfalls mit Anibiotikazusatz, reanimiert bzw. die getrockneten Membranen in hypotonen Lösungen zum Quellen gebracht und dann vor der Verwendung in isotone Lösungen überführt.

Die aus Nabelschnur präparierten Blutgefäße und das embryonale Bindegewebe werden auf entsprechende Weise konserviert.

Bevorzugte Anwendungsgebiete in der Chirurgie sind für die Häute das Über- oder Abdecken brüchiger Gewebe zur Vorbeugung von Nahtinsuffizienz und von Verwachsungen, die Deckung von Haut- und Schleimhautdefekten, das Belegen bzw. Überziehen von Gelenkflächen.

Embryonales Bindegewebe wird hingegen zum Ausfüllen von Hohlräumen, auch in Knochen, und zum Unterfüttern, insbesondere bei kosmetischen und plastischen Operationen, verwendet.

Die foetalen Blutadern dienen zur Überbrückung von Defekten an Blutgefäßen in der Gefäß-Chirurgie.

Die Fixierung der Transplantate erfolgt durch Biokleber und Situationsnähte. Als Biokleber werden vorzugsweise solche aus Fibrinogen/Thrombin oder aus verflüssigtem Kollagen oder Elastin mit Zusatz eines Verfestigers verwendet. Embryonales Bindegewebe kann in Form von Trockenpulvern mit Bioklebern vermischt appliziert werden.

Eine weitere Art der Vorbereitung lebenden Ausgangsgewebes ist die Adaptation der Gewebezellen an artspezifische oder individual-spezifische Histokompatibilitäts-Antigene der späteren Empfänger. Diese erfolgt durch Zusatz von Organextrakten zu den Überlebenskulturen der Gewebeexplantate. Es werden dabei zur Anpassung von tierischen Ausgangspräparaten an den Menschen wässrige Verdünnungen makromolekularer Organextrakte (DBP 1090821 und Europäisches Patent Veröffentlichungs-Nr. 0029 893A2) in ansteigenden Konzentrationen, von pg bis µg/ml Kulturmedium aus humanen Geweben, z.B.

Haut, Bindegewebe oder Blutzellen (Leukozyten) während einiger Tage, bis zur Verwendung, zugesetzt. Eine individuelle Anpassung kann durch Verwendung entsprechender Extrakte, die vom späteren Empfängerorganismus gewonnen werden, erfolgen. Zur Adaptation können auch Extrakte aus humanen Zellkulturen verwendet werden. Dieses Verfahren ist auch zur Anpassung von Zellkulturen von xenogenen oder allogenen Zellen an den späteren Empfänger geeignet. Auch hier können Wirkstoffe verwendet werden, die nach dem Verfahren zur Gewinnung von biologischen Wirkfaktoren des Europäischen Patentes, Veröffentlichungs-Nr. 0029 893A2, gewonnen werden. Das Verfahren eignet sich auch zur Adaptation von adultem Gewebe und von Gewebezellen.

Die weiteren Merkmale und Vorteile von bevorzugten Anwendungsformen der Erfindung ergeben sich aus den nachfolgenden Beispielen.

Beispiel 1

Von durch Sectio gewonnenen Plazenten vom Menschen, bzw. aus unmittelbar nach der Schlachtung entnommenen Tragsäcken von Tieren, z.B. vom Rind oder Schaf, werden unter sterilen Kautelen, die Eihäute und der nach Abnabelung zurückbleibende Rest der Nabelschnur in einer auf pH 7,2-7,35 gepufferten Ringerlösung mit Antibiotikazusatz (siehe Karl. F. Bauer und B. Mauersberger), während einiger Minuten, gegebenenfalls wiederholt, gespült. Danach werden die Eihäute entfaltet, auf geeignete Größe zugeschnitten und ohne Zug auf einen, in der Größe variablen Rahmen aufgelegt. In gleicher Weise können mehrere Häute übereinander gelegt werden.

Die Nabelschnur wird präparativ aufbereitet, längs gespalten und die in ihr enthaltenen Gefäße und mesenchymalen Anteile von der äußeren Haut entfernt und getrennt verarbeitet. Die äußere Oberfläche wird in gleicher Weise wie die Eihäute plan ausgebreitet, unter entsprechenden sterilen Bedingungen fixiert und mit physiologischer Lösung mit Antibiotikazusatz gespült. Die beschickten Rahmen werden in einem geeigneten Gefäß oder Kunststoffbeutel in Tyrode-Lösung oder Nährmedium mit Antibiotikazusatz gehalten und gekühlt auf +5° bis 10°C zum Ort der Verwendung transportiert. Die Verwendung soll innerhalb einer Woche, nach vorheriger Wiedererwärmung auf Körpertemperatur und Spülung in erneuertem Medium stattfinden.

## Beispiel 2

Die im Rahmen fixierten Eihäute werden auf einer Seite ohne Beeinflussung der gegenüberliegenden Seite mit einer Trypsin-Lösung folgender Zusammensetzung während 3-5 Minuten behandelt:

| | |
|---|---|
| Trypsin | 1,25g |
| Natriumzitrat | 1,48g |
| NaCl | 3,0 |
| $H_2O$ | 500,0 ml |
| pH | 7,8 |
| Temperatur | 37°C |

Danach wird diese Seite mit Ringer-Lösung zweimal hintereinander abgespült. Man läßt dann während 3 Minuten eine 0,1%ige Kollagenase-Lösung einwirken und spült wiederholt. Anschließend werden die Gewebe in einem künstlichen Nährmedium mit Antibiotikazusatz, in der Art von Gewebeexplantaten, vital-konserviert.

## Beispiel 3

Es wird allein mit 2%iger Perchlorsäure eine Seite der Eihäute während 5 Minuten behandelt und dann wiederholt gespült, bevor die Eihäute vital-konserviert werden.

## Beispiel 4

Es wird das aus Nabelstrang frei präparierte embryonale Bindegewebe in flüssigem Stickstoff schlagartig tiefgefroren und in gefrorenem Zustand pulverisiert und gefriergetrocknet. Gleichzeitig kann entsprechend dem Teilverfahren der Europäischen Patentanmeldung Nr. 80106066.6, während des Trocknungsvorganges eine Sulfatierung bzw. Sterilisierung gegen Viren erfolgen. Das erhaltene Trockenpulver wird kurz vor seiner Verwendung mit 0,5%iger NaCl-Lösung zum Quellen gebracht und mit einem Biokleber aus Fibrinogen/Thrombin vermischt. Der Brei wird zum Ausfüllen einer Knochenzyste verwendet.

## Beispiel 5

Die aus Nabelstrang frei präparierten Gefäße werden, unter Zusatz von DMSO in Tyrode-Lösung, während 3-5 Minuten gespült und anschließend stufenweise über flüssigem Stickstoff tiefgekühlt. Die Auftauung erfolgt vor der Verwendung in Nähr-Lösung, nach den Grundsätzen der Auftauung von tiefgefrorenen Gewebezellen aus der Zell- oder Gewebebank.

## Beispiel 6

Die nach Beispiel 2 bearbeiteten Eihäute werden wie im Beispiel 4 mit Dämpfen von konzentrierter Schwefelsäure im Vakuum bei Raumtemperatur behandelt und gleichzeitig getrocknet. Vor der Verwendung werden die Häute in 0,5%iger Na-Cl-Lösung zum Quellen gebracht und anschließend in 7,3 pH-gepufferter Ringer-Lösung, vor der Verwendung, gespült.

## Beispiel 7

Von Tieren gewonnene Eihäute sollen nach Mazeration einer Epithelschicht für die Transplantation auf den Menschen angepaßt werden, um etwaigen Abstoßungsreaktionen vorzubeugen. Die noch vitalen Membranen werden, wie im Beispiel beschrieben, in Überlebenskulturen vital-konserviert, indem man sie in einem geeigneten Nährmedium hält. Es ist zweckmäßig, zur Adaptation ein Minimalnährmedium zu verwenden (vgl. Paffenholz V. und K. Theurer: Einfluß von makromolekularen Organsubstanzen auf menschliche Zellen in vitro, I. Diploide Kulturen: Der Kassenarzt, Heft 27/1978 und Morbus Duchenne - die Beeinflussung zytoplasmatischer Enzyme in Zellkulturen von Patienten mit Muskeldystrophie: Der Kassenarzt 20, Heft 12/1980).

Den Nährmedien, z.B. auf der Basis von Hanks-Lösung, werden frische oder konservierte Organ- bzw. Zellextrakte aus durch Biopsie entnommenem, gesundem menschlichem Gewebe oder aus in Kultur gezüchteten foetalen oder jugendlichen menschlichen Zellen zugesetzt, in Konzentrationen zwischen pg bis µg/ml Nährmedium. Gegebenenfalls kann der Organextrakt dem gleichen Medium, beispielsweise bei permanenten Kulturen, wiederholt zugesetzt werden, sonst aber beim Umsetzen der Kultur. Es werden dabei insbesondere Extrakte aus foetalen und bzw. oder juvenilen Geweben aus Haut, Schleimhäuten, Mesenchym, parenchymatösen Organen oder aus Blutzellen, insbesondere Leukozyten, verwendet.

Zur individuellen Anpassung werden entsprechende Extrakte aus Geweben des späteren Empfängers verwendet. Die adaptierten Gewebe können dann, nach den in der Beschreibung genannten Methoden, bis zur Verwendung konserviert werden.


Beispiel 8


Das Verfahren zur adaptativen Anpassung in Kultur gehaltenen Zellen, wird auf allogene und xenogene Zellkulturen aus Organgeweben übertragen.

Beispiel 9

Das Verfahren von Beispiel 7 und 8 wird zur Adaptation von Organtransplantaten verwendet. Diese werden zusätzlich über das Gefäßsystem mit entsprechenden Medien perfundiert. Zusätzlich können $O_2$-übertragende Faktoren mitverwendet werden.

Anmelder: Prof. Dr.med. Karl Eugen Theurer
Dr.med. Karl Georg Theurer
Brunnwiesenstraße 23
D-7302 Ostfildern 1 (Ruit)
Bundesrepublik Deutschland

Verwendung von Eihäuten und Nabelschnur in nicht-bearbeitetem oder bearbeitetem Zustand.

Patentansprüche:

1. Verwendung von Eihäuten und Anteilen von Nabel-schnur, allogener und xenogener Herkunft in nativem, wie auch bearbeitetem Zustand bei chirurgischen Operationen, insbesondere zur Verstärkung brüchiger Gewebe durch Über- oder Abdecken, zur Vorbeugung von Nahtinsuffizienz und von Verwachsungen, zur Deckung von Haut- und Schleimhautdefekten, zum Ausfüllen und Unterfüttern von Hohlräumen, wie auch zum Belegen von Gelenkflächen und als Gefäßersatz.

2. Verfahren nach Anspruch 1, dadurch gekennzeichnet, daß die Epithelzellen der späteren Auflageseite enzymatisch und bzw. oder chemisch entfernt werden.

3. Verfahren nach Anspruch 1 zur Bearbeitung von Nabelschnur, dadurch gekennzeichnet, daß embryonales Bindegewebe, Blutgefäße und äußere Haut präparativ getrennt werden.

4. Verfahren nach Anspruch 1-3 zur Konservierung, dadurch gekennzeichnet, daß diese

a) in Art der Überlebenskulturen von Organexplantaten

b) durch Gefrieren bei tiefen Temperaturen unter Verwendung von Gefrierschutzmitteln z.B. Glyzerin oder DMSO

c) durch Gefriertrocknung

d) durch Gefriertrocknung und gleichzeitige partielle Sulfatierung durch Bedampfung mit Schwefelsäuredämpfen im Vakuum bei Raumtemperatur erfolgt, wobei die Membranen in einem durch elastische Züge oder Federn sich der Verkleinerung anpassenden Rahmen ausgespannt werden.

5. Verfahren nach Anspruch 1, dadurch gekennzeichnet, daß zur Fixierung Biokleber, z.B. aus Fibrin, Kollagen oder Elastin, verwendet werden.

6. Verfahren nach Anspruch 1 und 3 zur Anwendung von embryonalem Bindegewebe, dadurch gekennzeichnet, daß das daraus gewonnene Trockenpulver, vermischt mit Bioklebern, zur Ausfüllung von Hohlräumen verwendet wird.

7. Verfahren nach Anspruch 1., 3. und 4. zur Verwendung foetaler Blutadern, dadurch gekennzeichnet, daß diese zur Überbrückung von Gefäßdefekten verwendet werden.

0070328

8'. Verfahren nach Anspruch 1. und 4. zur Adaptation von in Überlebenskulturen gehaltenen Geweben und in Kultur gezüchteten Zellen an die Art- oder Individualspezifität des späteren Empfängers, dadurch gekennzeichnet, daß den Kulturmedien allogene oder individuelle Organ- oder Zellextrakte vom späteren Empfänger zugesetzt werden.

9. Verfahren nach Anspruch 8. zur Adaptation von vorgesehenen Organtransplantaten, dadurch gekennzeichnet, daß zusätzlich zur äußeren Benetzung eine Perfusion durch das Gefäßsystem erfolgt.

10. Verfahren nach Anspruch 8. und 9., dadurch gekennzeichnet, daß die Konservierung nach dem Verfahren des Anspruchs 4. erfolgt.

**EUROPÄISCHER TEILRECHERCHENBERICHT,**
der nach Regel 45 des Europäischen Patentübereinkommens für das weitere Verfahren als
europäischer Recherchenbericht gilt

Europäisches
Patentamt

0070328

Nummer der Anmeldung

EP 81 10 5731

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X,Y | US - A - 3 196 075 (IRENE NEUHAUSER)<br><br>* Spalte 3, Zeilen 33-43; Spalte 8, Zeilen 23-28; Spalte 13, Zeile 49 bis Spalte 14, Zeile 25 *<br><br>--- | 2,4,5 |
| X,Y | FR - M - 7 205 (ADAM GRAETZ)<br><br>* Seite 6, Zusammenfassungen 1-6; Seite 2, Linke Spalte, Zeile 38 bis rechte Spalte, Zeile 42 *<br><br>--- | 2-7 |
| X,Y | US - A - 3 894 530 (I. DARDIK et al.)<br><br>* Ansprüche; Spalte 1, Zeile 64 bis Spalte 2, Zeile 7 *<br><br>--- | 3-7 |
| Y | DE - A - 2 944 278 (K. THEURER)<br><br>* Ansprüche und Beispiel 1 *<br><br>--- | 4 |
| Y | FR - A - 1 473 468 (J. TANNER)<br><br>* Seite 7, Zusammenfassung, Punkte 13,14 *<br><br>--- | 4<br><br>./. |

**KLASSIFIKATION DER ANMELDUNG (Int. Cl.³)**

A 61 L  15/04
A 61 F   1/00
A 01 N   1/02
A 61 K  35/12
C 12 N   5/00

**RECHERCHIERTE SACHGEBIETE (Int. Cl.³)**

A 61 L
A 61 L
A 01 N
A 61 K
C 12 N

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:
Unvollständig recherchierte Patentansprüche:
Nicht recherchierte Patentansprüche:
Grund für die Beschränkung der Recherche:

Die Nachforschung hatte Bezug auf die Massnahmen gemäss den Patentansprüchen 1-10, an und für sich

Verfahren zur chirurgischen oder therapeutischen Behandlung des menschlichen oder tierischen Körpers (sie Art. 52(4) des Europäischen Patentübereinkommens)

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| Recherchenort | Bdatum der Recherche | Prüfer |
|---|---|---|
| Den Haag | 8.April 1982 | RIJCKEBOSCH |

EPA Form 1505.1  06.78

Europäisches
Patentamt
**EUROPÄISCHER TEILRECHERCHENBERICHT**

0070328
Nummer der Anmeldung

EP 81 10 5731
-2-

| EINSCHLÄGIGE DOKUMENTE | | | KLASSIFIKATION DER ANMELDUNG (Int. Cl. ) |
|---|---|---|---|
| Kategorie | Kennzeichnung des Dokuments mit Angabe, soweit erforderlich der maßgeblichen Teile | betrifft Anspruch | |
| Y | US - A - 4 167 945 (S. GOTTLIEB) | | |
| | * Ansprüche 1-3; Spalte 3, Zeilen 1-8 und 12-24 * | 5 | |
| | --- | | |
| Y | US - A - 2 621 145 (M. SANO) | | |
| | * Anspruch 1 * | 5,6 | |
| | --------- | | **RECHERCHIERTE SACHGEBIETE** (Int. Cl.³) |